# EUROPÄISCHE PATENTSCHRIFT

(11) **EP 2 846 951 B1**
(45) Veröffentlichungstag und Bekanntmachung des Hinweises auf die Patenterteilung: **21.03.2018**
(21) Anmeldenummer: 13736783.5
(22) Anmeldetag: 03.05.2013
(51) Int. Cl.: B23B 31/107, A61B 17/16

(54) **SCHNELLSPANN-KUPPLUNG**
QUICK-ACTION COUPLING
RACCORD À SERRAGE RAPIDE

(30) Priorität: 08.05.2012 DE 102012207651
(43) Veröffentlichungstag der Anmeldung: 18.03.2015
(73) Patentinhaber: Aesculap AG, 78532 Tuttlingen (DE)
(72) Erfinder: STERN, Andreas, 78087 Mönchweiler (DE)
(74) Vertreter: Koller, Tobias Kilian
(86) Internationale Anmeldenummer: PCT/EP2013/059293
(87) Internationale Veröffentlichungsnummer: WO 2013/167496

(56) Entgegenhaltungen:
- EP-A1- 0 056 266
- US-A- 3 367 727
- US-A- 5 013 194
- US-A- 5 222 848
- US-A1- 2007 264 093

## Beschreibung

Die vorliegende Erfindung betrifft eine Schnellspann-Kupplung gemäß dem Oberbegriff des Anspruchs 1 für ein chirurgisches Drehwerkzeug wie beispielsweise einen Knochenbohrer oder eine Knochenfräse. Eine Kupplung mit solchen Merkmalen ist beispielsweise aus US 5,222,848 bekannt. Bei Operationen an den Knochen eines Patienten muss ein Knochen oft gebohrt oder gefräst werden. Hierbei muss häufig das verwendete Drehwerkzeug während der Operation (OP) ausgewechselt werden, sei es verschleißbedingt oder da ein Bohrer mit anderem Durchmesser oder eine Fräse mit einer anderen Oberfläche eingesetzt werden soll. Im OP-Umfeld herrscht ein großer Kostendruck, sodass es wichtig ist, dass ein Werkzeugwechsel schnell und sicher ausgeführt werden kann. Sollte sich ein Werkzeugwechsel verzögern, betrifft dies nicht nur den Bediener des Gerätes sondern das gesamte Personal im Operationssaal, welches dann auf den Bediener des Gerätes warten muss.

Im klinischen Bereich werden an Werkzeuge aber weitaus größere Anforderungen gestellt als an vergleichbare Werkzeuge zum Beispiel im Baubereich. Die Bediensicherheit muss zwar in allen Umfeldern gegeben sein, jedoch stört es auf einer Baustelle nicht, wenn beim Einsetzen eines Bohrers in eine Kupplung dieser zunächst einmal auf den Boden fällt. Im klinischen Bereich ist stets auf Sterilität zu achten, sodass ein Werkzeug, welches auf den Boden fällt oder in einen anderen nicht als steril angesehenen Bereich (üblicherweise alle Flächen und Bereiche, welche sich unterhalb des OP-Tuchs befinden) eindringt, durch ein steriles Werkzeug ersetzt werden. Dies führt zu Verzögerungen im OP-Ablauf und zu erhöhten Wiederaufbereitungskosten der Werkzeuge, da nach der Operation beide Werkzeuge gereinigt und sterilisiert werden müssen.

Darüber hinaus muss auch der Werkzeugträger gereinigt und sterilisiert werden können, um einen Mehrfachbetrieb zu gestatten. Der Werkzeugträger ist hier beispielsweise die Bohrmaschine oder die Fräsmaschine. Da die Kupplung ein Teil des Werkzeugträgers ist, muss diese also ebenfalls für eine Reinigung und Sterilisierung geeignet sein. Hierzu können weitere Anforderungen an die Werkzeugkupplung auftreten.

### Stand der Technik

In der europäischen Patentanmeldung EP 0 056 266 A1 ist ein Schnellwechselfutter offenbart. Deses Schnellwechselfutter weist eine Drehwelle auf, in deren distalen Ende eine Werkzeugaufnahme ausgebildet ist, welche mit einem einzuführenden Werkzeug einen solchen Formschluss herstellt, dass das Werkzeug in der Werkzeugaufnahme nicht drehbar ist. Üblicherweise sind die Aufnahmebereiche der Werkzeuge dazu sechseckig ausgebildet. Ein pilzförmiger Sperrkörper ist in einer radialen Durchbrechung der Mantelfläche der Werkzeugaufnahme aufgenommen und zur Drehachse der Werkzeugaufnahme hin vorgespannt, sodass er, wenn ein Werkzeug in die Aufnahme eingeführt wird, in eine an dem Werkzeug ausgebildete umlaufende Nut eintritt. Durch eine drehbare Hülse, die durch eine Feder vorgespannt ist, wird die Position des Sperrkörpers gesichert, sodass das Werkzeug fest in der Werkzeugaufnahme gehalten ist, solange die Hülse nicht gegen die Vorspannung der Feder in eine Öffnungsstellung gedreht wird. Dieses Schnellwechselfutter besteht jedoch aus einer Vielzahl von Bauteilen, welche nach ihrem Zusammenbau unzerlegbar miteinander verbunden sind. Zudem sind mehrere Hohlräume (z.B. Federgehäuse) und Schlitze ausgebildet, in denen sich Gewebeteile und Körperflüssigkeiten sammeln könnten und welche im Rahmen eines klinischen Sterilisationsablaufs nicht sicher gereinigt und sterilisiert werden können. Dieses Schnellwechselfutter ist daher keinesfalls für ein chirurgisches Drehwerkzeug geeignet.

In dem US Patent US 5,013,194 ist ein Werkzeugfutter gezeigt, welches ebenfalls ein sechseckiges und mit einer umlaufenden Nut versehenes hinteres Ende eines Werkzeuges aufnehmen kann. Dieses Werkzeug weist zwei Kugeln als Sperrkörper auf, die sich im der Verriegelungsstellung an einer vorstehenden Innenfläche einer verschiebbaren Hülse abstützen. Die Kugeln können in der Freigabestellung der Hülse in einen ringförmigen Raum eintreten, der in der Hülse ausgebildet ist. Eine Feder, welche die Hülse in die Verriegelungsstellung vorspannt, ist in einem zweiten ringförmigen Raum in der Hülse angeordnet. Die Feder stützt sich an einer Seitenwand des Stützvorsprungs und an einem Sprengring ab, welcher in eine Nut der Drehwelle eingreift. Dieses Werkzeugfutter ist ebenfalls nicht zerlegbar und somit nicht sterilisierbar. Ein solches Werkzeugfutter kann nicht für ein chirurgisches Drehwerkzeug verwendet werden.

Die Aufgabe der vorliegenden Erfindung ist es daher, eine Schnellspann-Kupplung bereit zu stellen, die schnell, einfach und sicher zu betätigen ist und zudem für einen klinischen Reinigungs- und Sterilisiervorgang geeignet ist. Eine weitere Aufgabe der vorliegenden Erfindung ist es, einen Werkzeugträger mit einer solchen Schnellspann-Kupplung bereit zu stellen.

Die Aufgabe der vorliegenden Erfindung wird durch eine Schnellspann-Kupplung nach Anspruch 1 gelöst. Vorteilhafte Weiterentwicklungen der Erfindung sind Gegenstand der abhängigen Ansprüche.

Gemäß einem Aspekt der vorliegenden Erfindung wird eine Schnellspann-Kupplung für ein chirurgisches Drehwerkzeug geschaffen mit einer Drehwelle, in deren distaler Endfläche eine Aufnahme ausgebildet ist. Diese Aufnahme ist daran angepasst, ein chirurgisches Drehwerkzeug drehfest aufzunehmen. Dies wird gewöhnlich dadurch erreicht, dass das hintere ende des Werkzeugs und dementsprechend die Innenwandfläche der Aufnahme mit einem mehreckigen und vorzugsweise sechseckigen Querschnitt ausgebildet sind. Die Drehwelle weist im Bereich ihres distalen Endes mindestens einen radialen Vorsprung und mindestens eine radiale Durchbrechung auf, wobei in der mindestens einen Durchbrechung ein Sperrkörper angeordnet ist. Der Sperrkörper ist dabei in radialer Richtung zu der Drehwelle bewegbar gehalten.
Die Schnellspann-Kupplung weist zudem eine Hülse auf, die mit ihrem proximalen Ende über das distale Ende der Drehwelle schiebbar ist. Dabei weist die Innenfläche der Hülse einen Bereich auf, in dem der Innendurchmesser des proximalen Abschnitts gegenüber dem Innendurchmesser des distalen Abschnitts verkleinert ist. Die Innenfläche der Hülse kann noch weitere Bereiche aufweisen, die zum Beispiel eine Anlagefläche für das unten genannte elastische Element ausbilden, aber der hier definierte Bereich ist daran angepasst, bei einer Bewegung in distaler Richtung in Anlage an den mindestens einen Sperrkörper zu gelangen und ihn zur Achse der Drehwelle hin zu drängen. Bei einer entgegen gesetzten Bewegung der Hülse in proximaler Richtung wird der mindestens eine Sperrkörper frei gegeben, sodass sich dieser zumindest so weit radial nach außen bewegen kann, dass dessen inneres Ende aus der Werkzeugaufnahme austritt. Die Hülse weist mindestens einen Schlitz auf, der einen ersten Schlitzabschnitt und wenigstens einen weiteren Schlitzabschnitt aufweist, wobei sich der erste Schlitzabschnitt vom proximalen Ende der Hülse aus erstreckt und der weitere Schlitzabschnitt mit dem ersten Schlitzabschnitt in Verbindung steht. Vorzugsweise bildet der weitere Schlitzabschnitt mit dem ersten Schlitzabschnitt einen Winkel α von mindestens 90°. Der weitere Schlitzabschnitt kann der zweite, dritte oder auch ein anderer Schlitzabschnitt sein und kann sich sogar aus mehreren von diesen Schlitzabschnitten zusammensetzen. Dass sich der erste Schlitzabschnitt von dem proximalen Ende der Hülse aus erstreckt bedeutet, dass er sich zum proximalen Ende der Hülse hin öffnet, sodass der zugehörige Vorsprung an der Drehwelle von dem proximalen Ende der Hülse her in diesen Schlitz eingeführt werden kann. Der Schlitz kann auch als Nut ausgebildet sein, welche an der Innenwand der Hülse vorgesehen ist, sodass der Schlitz und die einzelnen Schlitzabschnitte von außen nicht erkennbar sind. Darüber hinaus weist die Schnellspann-Kupplung ein elastisches Element auf, welches die Hülse relativ zu der Drehwelle in distaler Richtung drängt.

Mit einer solchen Ausgestaltung der Schnellspann-Kupplung ist es möglich, die Schnellspann-Kupplung in wenigstens zwei Einzelteile zu zerlegen, nämlich die Hülse und die Werkzeugaufnahme, wobei das elastische Element entweder Teil eines der beiden anderen Teile sein kann, an einem der beiden anderen Teile fest montiert sein kann, oder als einzelnes drittes Bauteil vorgesehen sein kann. Auf diese Weise kann die Schnellspann-Kupplung für eine Reinigung und Sterilisation leicht und schnell zerlegt und anschließend wieder zusammen gebaut werden, sodass die Schnellspann-Kupplung während der Reinigung und Sterilisation keine Hohlräume und enge Schlitze aufweist, die schlecht zu reinigen und zu sterilisieren sind. Gleichzeitig wird die Anzahl der Bauteile maximal verringert, sodass keine aufwändige Montage und Demontage erfolgen muss. Zusätzlich dazu wird für die Montage und Demontage keinerlei Werkzeug benötigt.

Gemäß einer vorteilhaften Weiterentwicklung der vorliegenden Erfindung kann vorgesehen sein, dass der weitere Schlitzabschnitt mit dem ersten Schlitzabschnitt einen Winkel von mindestens 90° bildet. In diesem Fall muss die Hülse zumindest gegen die Reibkraft um ihre Achse gedreht werden, die zwischen weiteren Schlitzabschnitt und seitlichem Vorsprung an der Werkzeugaufnahme ausgebildet ist und von dem elastischen Element hervorgerufen wird, um in eine Position zu gelangen, in der die Hülse von der Werkzeugaufnahme abnehmbar ist. Bei einem Winkel von mehr als 90° muss das elastische Element zudem gestaucht werden, damit eine Drehung der Hülse um deren Achse möglich ist.

Gemäß einer vorteilhaften Ausgestaltung der vorliegenden Erfindung ist das elastische Element eine Spiralfeder, welche um die Drehwelle herum angeordnet ist. Vorteilhafter Weise wird diese durch die Hülse im montierten Zustand vollständig bedeckt. Spiralfedern sind bekannte Bauteile in chirurgischen Geräten, sodass kein Zweifel an der Reinigbarkeit und Sterilisierbarkeit der Spiralfeder besteht. Zudem sind ausreichend geeignete Materialien bekannt, um eine solche Spiralfeder auszubilden. Durch die Anordnung der Spiralfeder außerhalb der Werkzeugaufnahme kann die Länge der Schnellspann-Kupplung minimiert werden. Bei einer Anordnung der Feder im Inneren der Werkzeugaufnahme, was prinzipiell möglich ist, müsste sonst in der Werkzeugaufnahme der Bereich, der für den Formschluss mit dem Werkzeug zuständig ist, proximal zu dem Bereich angeordnet sein, in dem die Feder angeordnet ist. Bei einer äußeren Anordnung der Spiralfeder können die Spiralfeder und der Formschluss mit dem Werkzeug in demselben axialen Bereich erfolgen.

Gemäß einer weiteren vorteilhaften Ausgestaltung der vorliegenden Erfindung ist an der Hülse und/oder an der Drehwelle eine Anlagefläche für das elastische Element ausgebildet. Alternativ dazu kann das elastische Element auch so ausgebildet sein, dass es auf die Außenfläche der Werkzeugaufnahme gepresst wird. Die Kraft, die das elastische Element auf die Hülse aufbringen muss, ist nicht sehr groß. Würde das elastische Element auf die Außenfläche der Werkzeugaufnahme gepresst, wäre dies noch immer ohne Werkzeug möglich und das elastische Element könnte auch ohne Werkzeug wieder von der Werkzeugaufnahme abgezogen werden. Besser ist es jedoch, wenn eine Anlagefläche an der Werkzeugaufnahme vorgesehen ist, sodass sich das elastische Element quasi kraftfrei von der Werkzeugaufnahme lösen lässt. Alternativ kann das elastische Element fest an der Werkzeugaufnahme angeschlossen sein, z.B. indem es angeschweißt ist. In diesem Fall kann das elastische Element nicht von der Werkzeugaufnahme abgehen, sodass es nicht verloren gehen kann.

Gemäß noch einer weiteren vorteilhaften Ausgestaltung der vorliegenden Erfindung ist der Sperrkörper eine Kugel und die Durchbrechung weist an beiden Seiten einen gegenüber einem dazwischen liegenden Abschnitt verkleinerten Durchmesser auf. Eine Kugel eignet sich besonders als Sperrkörper, da sie erstens in jeder beliebigen Lage eingebaut werden kann, was den Einbauvorgang erleichtert, und zweitens sich in der Durchbrechung drehen kann, was die Reibung verringert, wenn ein Werkzeug in die Werkzeugaufnahme eingeführt wird und dabei in Kontakt mit dem Sperrkörper kommt. Letzteres gilt auch für den Kontakt zwischen Sperrkörper und Anlagefläche in der Hülse, also dem Bereich der Innenfläche der Hülse, an dem der Durchmesser verkleinert ist. Alternativ kann auch ein zylindrischer, kegelförmiger oder pilzförmiger Sperrkörper vorgesehen sein. Es muss aber auch kein rotationssymmetrischer Körper sein. Vorzugsweise ist der Sperrkörper aber rotationssymmetrisch und auf beiden Seiten, d.h. zum Werkzeug und zur Anlagefläche hin, mit einer abgerundeten Oberfläche versehen, wie dies insbesondere die Kugel zeigt.

Gemäß einer anderen vorteilhaften Ausgestaltung der vorliegenden Erfindung erstreckt sich der erste Schlitzabschnitt im Wesentlichen in distaler Richtung und dieser erste Schlitzabschnitt steht an seinem distalen Ende mit dem zweiten Schlitzabschnitt in Verbindung, der sich im Wesentlichen in radialer Richtung erstreckt. An dieser Stelle wird zunächst auf das Grundprinzip der verschiedenen Schlitzabschnitte eingegangen.

Die Schlitzabschnitte müssen zusammen drei verschiedene Aufgaben erfüllen, nämlich (1) den Zugang für den Zapfen bilden, (2) die Schnellspann-Kupplung gegen ein unbeabsichtigtes Zerlegen sichern und (3) eine Verschiebung der Hülse zwischen der Freigabeposition und der Verriegelungsposition für das eingesetzte Werkzeug gewährleisten. Zusätzlich dazu kann noch die Aufgabe (4) erfüllt werden, die Hülse in der Freigabeposition und/oder der Verriegelungsposition vorrübergehend zu sperren. Insgesamt werden diese drei oder vier Aufgaben von den verschiedenen Schlitzabschnitten erfüllt, wobei ein Schlitzabschnitt auch mehrere Aufgaben gemeinsam erfüllen kann und sich eine Aufgabe auf mehrere Schlitzabschnitte erstrecken kann. Die Aufgabe (1) aber wird immer durch den ersten Schlitzabschnitt erfüllt, da der erste Schlitzabschnitt als der Schlitzabschnitt definiert ist, der sich zum proximalen Ende der Hülse hin öffnet. Wichtig ist hierbei, dass die Aufgabe (1) und die Aufgabe (3) nicht von demselben Schlitzabschnitt übernommen wird, da dann nicht sicher gestellt werden kann, dass die Schnellspann-Kupplung nicht doch unbeabsichtigt zerlegt wird. Die Aufgabe (2) kann von einem eigenen Schlitzabschnitt erfüllt werden oder von einem Schlitzabschnitt, der alleine oder zusammen mit einem anderen Schlitzabschnitt die Aufgabe (1) oder (3) und/oder ggf. auch Aufgabe (4) erfüllt.

Die verwendeten Richtungsangaben sind im Folgenden erklärt. Die distale Richtung ist die Richtung zu der Werkzeugspitze hin und ist entgegengesetzt zu der proximalen Richtung, die zu dem Werkzeugträger hin verläuft. Die radiale Richtung ist rechtwinklig zu der proximalen und distalen Richtung und verläuft entlang des Umfangs der Werkzeugaufnahme und der Hülse. Die radiale Richtung bezeichnet allgemein beide möglichen Umlaufrichtungen. Die gegenradiale Richtung ist die radiale Richtung, die einer zuvor genannten Richtung gegengleich ist, also quasi in umgekehrter Drehrichtung verläuft. Wenn ein Schlitzabschnitt radial und distal bzw. proximal verläuft, dann verläuft er schräg entlang der Hülse. Alle Richtungsangaben beziehen sich auf die Wesentliche Richtung und erlauben durchaus kleinere Abweichungen, die die Funktionalität nicht beeinflussen. Bei gebogenen oder gekrümmten Schlitzen ist die Richtung die kürzeste Verbindung beider Enden eines Schlitzabschnitts auf der Mantelfläche der Hülse. Durch die Zylinderform der Hülse sind alle Schlitze außer rein distal oder proximal verlaufenden Schlitzen gekrümmt. Dies soll aber nicht durch den Begriff gekrümmte Schlitze bzw. gebogene Schlitze beschrieben werden. Diese Begriffe stehen vielmehr für Schlitze, die auch dann eine Krümmung oder Biegung aufweisen, wenn man die Mantelfläche der Hülse auf eine Ebene abwickelt. Demnach kann es auch mehrdimensional gekrümmte bzw. gebogene Schlitze geben. Außerdem können die Schlitzabschnitte auch stufenförmig ausgebildet werden, d.h. sie können aus distalen/proximalen und radialen/gegenradialen Abschnitten zusammengesetzt sein.

Nach der vorgenannten Ausgestaltung der Schnellspann-Hülse gibt es zahlreiche mögliche Anordnungen und entsprechende Aufgabenverteilungen für die einzelnen Schlitzabschnitte, die aber zusammen alle die Aufgaben (1) bis (3) erfüllen und somit eine funktionsfähige Schnellspann-Kupplung bilden.

Gemäß noch einer weiteren vorteilhaften Ausgestaltung der vorliegenden Erfindung erstreckt sich der erste Schlitzabschnitt schräg in distaler und radialer Richtung und steht an seinem distalen Ende mit dem zweiten Schlitzabschnitt in Verbindung. Der erste Schlitzabschnitt erfüllt hierbei die Aufgaben (1) und (2) und der zweite Schlitzabschnitt erfüllt die Aufgabe (3). Ein Freigeben bzw. Verriegeln des Werkzeugs (Aufgabe (1)) in der Werkzeugaufnahme erfolgt hier durch den zweiten Schlitzabschnitt und ein Zerlegen bzw. Zusammenbauen (Aufgabe (3)) durch den ersten Schlitzabschnitt. Das Sichern bzw. Entsichern gegen ein unbeabsichtigtes Zerlegen (Aufgabe (2)) erfolgt hier ebenfalls durch den ersten Schlitzabschnitt. Die Hülse muss zum Zerlegen und zum Zusammenbauen jeweils gedreht und gleichzeitig nach distal geschoben werden. Je nach Reibung und Stärke des elastischen Elements kann das Verschieben der Hülse nach distal auch von dem elastischen Element übernommen werden. Wenn der dritte Schlitzabschnitt stufenförmig ausgebildet ist, kann zum Beispiel verhindert werden, dass das Werkzeug versehentlich frei gegeben wird, wenn die Hülse an einem Objekt, bspw. Gewebe, in Anlage gerät. Die Hülse kann durch einfaches Drücken in proximaler Richtung von der Verriegelungsposition nur in eine Zwischenstellung gebracht werden, von wo aus zunächst eine radiale Verdrehung erforderlich ist, um dann die Möglichkeit zu haben, die Hülse durch weiteres Drücken in die proximale Richtung in die Freigabeposition zu bringen oder durch weiteres radiales Verdrehen und anschließendes distales Verschieben die Kupplung zu demontieren.

Gemäß noch einer weiteren vorteilhaften Ausgestaltung der vorliegenden Erfindung ist im ersten Schlitzabschnitt in der Nähre des proximalen Randes der Hülse eine radiale Stufe ausgebildet. Diese Stufe ist dazu vorgesehen, zu verhindern, dass die Hülse durch die Vorspannung des elastischen Elements von der Werkzeugaufnahme springt, wenn die Hülse beim Montieren oder Demontieren versehentlich losgelassen wird. Wird mit einem so ausgestalteten ersten Schlitzabschnitt die Hülse beim Montieren oder Demontieren versehentlich losgelassen, während sich der Vorsprung distal der radialen Stufe im ersten Schlitzabschnitt befindet, schlägt der Vorsprung an der radialen Stufe an und bleibt dort in Anlage. Das elastische Element kann zudem so gestaltet werden, dass in dieser Stellung nur noch eine sehr geringe Vorspannung auf die Hülse aufgebracht wird, sodass dann, wenn sich der Vorsprung proximal der radialen Stufe befindet, diese verbleibende Vorspannung nicht mehr ausreicht, um die Hülse von der Werkzeugaufnahme springen zu lassen.

Gemäß einer weiteren vorteilhaften Ausgestaltung der vorliegenden Erfindung sind zwei oder drei Schlitze an einer Hülse vorgesehen sind, die in gleichwinkligen Abständen entlang der Umfangsrichtung der Hülse angeordnet sind. Entsprechend ist eine gleiche Anzahl von radialen Vorsprüngen an der Drehwelle ausgebildet sind, die entsprechend angeordnet sind. Somit läuft jeder Vorsprung in einem Schlitz. Dadurch, dass die Schlitze und Vorsprünge in gleichen Winkelabständen angeordnet sind, gibt es keine falsche Anordnung der Hülse zu der Werkzeugaufnahme, solange nur ein einziger Vorsprung in einem Schlitz aufgenommen ist. Alternativ dazu kann man ungleiche Winkelabstände verwenden, um zum Beispiel die Zuordnung bestimmter Hülsen zu bestimmten Werkzeugaufnahmen sicher zu stellen.

Gemäß einer weiteren vorteilhaften Ausgestaltung der vorliegenden Erfindung sind zwei oder drei radiale Durchbrechungen in der Seitenwand der Werkzeugaufnahme ausgebildet sind und in jeder Durchbrechung ist eine Kugel als Sperrkörper angeordnet. Diese Kugelwird in der Durchbrechung gehalten, indem zumindest ein Teil wenigstens eines Rands der Durchbrechung zur Lochmitte hin plastisch verformt ist. Um die Reibung zwischen Rand und Kugel zu minimieren ist es sinnvoll, wenn zumindest annähernd der gesamte Rand entlang der Umfangsbegrenzung der Durchbrechung zu der Mitte der Durchbrechung hin plastisch verformt ist. Die plastische Verformung wird zum Beispiel durch einen hohlzylindrischen Stempel erzielt, der einen etwas größeren Durchmesser als die Durchbrechung aufweist und konzentrisch zur Durchbrechung in die Oberfläche der Werkzeugaufnahme gepresst wird. Da dieses Prinzip an der Innenweite der Durchbrechung aus Platzgründen schwer auszuführen ist, kann die Kugel nach innen hin dadurch in der Durchbrechung gehalten werden, dass die Durchbrechung gebohrt wird, wobei entweder zunächst ein kleines Durchgangsloch und anschließend ein konzentrisches und etwas größeres Sackloch gebohrt wird, oder durch einen Bohrer, der im Bereich seiner Spitze einen kleineren Durchmesser aufweist. Im Prinzip kann die Durchbrechung auch auf einer Seite oder auf beiden Seiten mit einem Gewinde versehen sein, in das ein Verengungsring eingeschraubt wird, der den Durchmesser der Öffnung der Durchbrechung verringert. Ein solcher Verengungsring kann auch anderweitig in die Durchbrechung eingebracht und fixiert bzw. an der Hülse montiert werden.

Gemäß einer weiteren vorteilhaften Ausgestaltung der vorliegenden Erfindung ist in dem Bereich der Innenfläche der Hülse zwischen dem proximalen Abschnitt und dem distalen Abschnitt eine Stufe oder eine geneigte Fläche vorgesehen. Eine Stufe hat den Vorteil, dass nur ein sehr kurzer Weg der Hülse in distaler/proximaler Richtung erforderlich ist, um von der Freigabeposition in die Verriegelungsposition der Schnellspann-Kupplung zu gelangen. Eine geneigte Fläche hat den Vorteil, dass keine Kraftspitze während der Verschiebung auftritt und die Hülse nicht kurzzeitig festhängt, wenn die Stufe an der oder den Sperrelementen aus seitlicher (d.h. axialer) Richtung anliegt. Andere Formen der Anlagefläche sind ebenfalls möglich, beispielsweise kann die Anlagefläche mehrstufig, paraboloid oder hyperboloid ausgebildet sein.

Gemäß einer weiteren vorteilhaften Ausgestaltung der vorliegenden Erfindung der Schlitz derart ausgestaltet ist, dass er sich über die gesamte Wanddicke der Hülse erstreckt. Prinzipiell kann der mindestens eine Schlitz auch als eine Nut ausgebildet sein, die an der Innenfläche der Hülse ausgebildet ist. Dies hat aber den Nachteil, dass der Verlauf der Schlitzabschnitte für den Benutzer nicht erkennbar ist, sodass eine Handhabung der Schnellspann-Kupplung erschwert ist. Diesen Nachteil kann man aber ausgleichen, indem man den Verlauf der Schlitzabschnitte auf der Außenseite der Hülse grafisch andeutet oder aufzeichnet. Damit erreicht man den Vorteil, dass der Benutzer nicht mit den Schlitzabschnitten und insbesondere deren Kanten in Berührung kommt.

Gemäß einer weiteren vorteilhaften Ausgestaltung der vorliegenden Erfindung ist das chirurgische Drehwerkzeug insbesondere ein Bohrer oder eine Fräse. Die vorliegende Erfindung kann aber auch für alle anderen Drehwerkzeuge verwendet werden.

Weitere Vorteile und Merkmale der Erfindung sind dem Fachmann aus den beigefügten Figuren und der detaillierten Beschreibung der Ausführungsbeispiele ersichtlich.
- Fig. 1A: ist eine seitliche Ansicht einer erfindungsgemäßen Schnellspann-Kupplung sowie eines geeigneten Werkzeugs während des Einführens des Werkzeugs in die Schnellspann-Kupplung gemäß einem ersten Ausführungsbeispiel;
- Fig. 1B: ist eine seitliche Ansicht einer Schnellspann-Kupplung nach Fig. 1A, in der ein Werkzeug verriegelt ist;
- Fig. 1C: ist eine seitliche Ansicht einer Schnellspann-Kupplung nach Fig. 1A, wobei die Schnellspann-Kupplung zerlegt ist;
- Fig. 1D: ist eine seitliche Schnittansicht entsprechend der Fig. 1A;
- Fig. 1E: ist eine seitliche Schnittansicht entsprechend der Fig. 1B;
- Fig. 1F: ist eine seitliche Schnittansicht entsprechend der Fig. 1C;
- Fig. 2: sind schematische seitliche Ansichten eines Ausschnitts der Hülsen gemäß zahlreicher Ausführungsbeispiele der vorliegenden Erfindung; und
- Fig. 3: sind schematische Schnittansichten der Hülsen gemäß zahlreicher Ausführungsbeispiele der vorliegenden Erfindung.

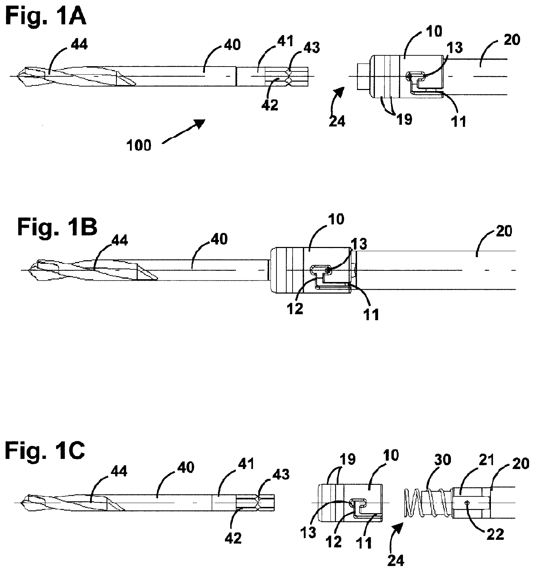

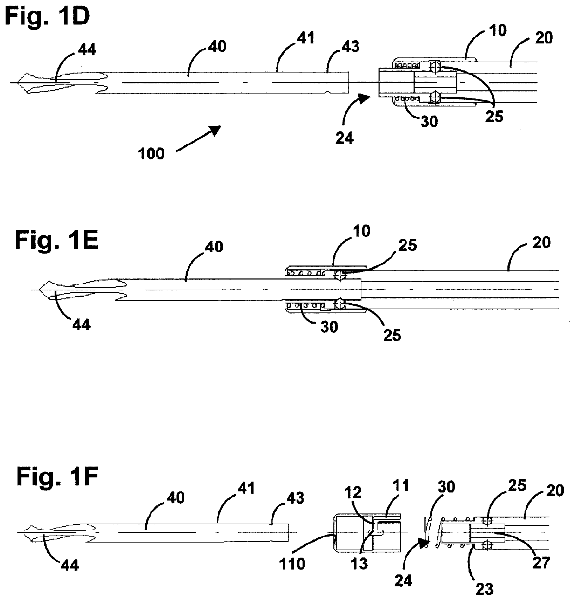

Ein erstes Ausführungsbeispiel der vorliegenden Erfindung ist im Folgenden unter Bezugnahme auf die Fig. 1 bis 3 im Detail beschrieben. Insbesondere ist dieses Ausführungsbeispiel unter Bezugnahme auf die Fig. 1, 2A und 3A beschrieben.

Eine Schnellspann-Kupplung 100 gemäß dem ersten Ausführungsbeispiel weist eine Drehwelle 20 auf, in deren distaler Endfläche eine Werkzeugaufnahme 24 ausgebildet ist. Die Werkzeugaufnahme 24 nimmt das hintere Ende 41 eines chirurgischen Drehwerkzeugs 40 drehfest auf. Dazu ist die Werkzeugaufnahme 24 an die sechseckige Querschnittsform des hinteren Endes 41 des Werkzeugs 40 angepasst, die in Fig. 1A und 1C gezeigt ist. Die einzelnen Flächen 27 der sechseckigen Werkzeugaufnahme 24 liegen dabei an den einzelnen Flächen 42 des Drehwerkzeuges an. Die Drehwelle 20 weist im Bereich ihres distalen Endes 21 einen radialen Vorsprung 22 und zwei radiale Durchbrechungen auf, wobei die Durchbrechungen in den Figuren 1A bis 1C nicht dargestellt sind. In den beiden Durchbrechungen ist je eine Kugel 25 als Sperrkörper angeordnet, die beide jeweils in radialer Richtung zu der Drehwelle 20 bewegbar sind und so in die umlaufende Nut 43 in dem hinteren Ende 41 des Werkzeugs 40 eintreten können, wenn sich das hintere Ende 41 des Werkzeugs 40 in der Werkzeugaufnahme 24 befindet.

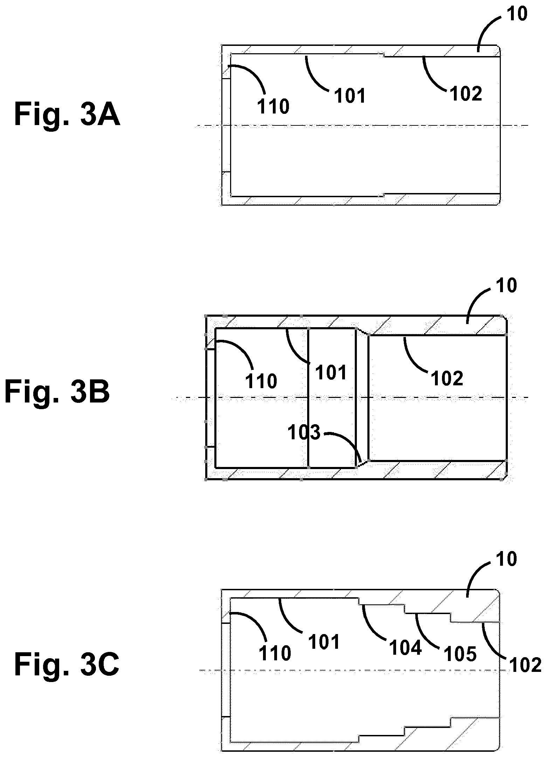

Die Schnellspann-Kupplung 100 weist zudem eine Hülse 10 auf, die mit ihrem proximalen Ende über das distale Ende 21 der Drehwelle 20 schiebbar ist. Die Innenfläche der Hülse 10 weist einen Bereich auf, in dem der Innendurchmesser des distalen Abschnitts 102 durch eine einzelne Stufe gegenüber dem Innendurchmesser des proximalen Abschnitts 101 vergrößert ist, wie dies in der Fig. 3A gezeigt ist. Aus den Fig. 1D bis 1F ist ersichtlich, dass die Hülse 10 einen im Wesentlichen konstanten Innendurchmesser aufweist. Lediglich eine umlaufende Nut an der Innenfläche der Hülse erzeugt die vorstehend beschriebene Stufe und bestimmt somit auch den definierten Bereich der Innenfläche. Die Hülse 10 weist zudem einen Schlitz 11, 12, 13 auf, der einen ersten Schlitzabschnitt 11, einen zweiten Schlitzabschnitt 12 und einen dritten Schlitzabschnitt 13 aufweist. Der Schlitz ist bei diesem Ausführungsbeispiel als durchgehender Schlitz ausgebildet. Der erste Schlitzabschnitt 11 erstreckt sich vom proximalen Ende der Hülse 10 aus. Am distalen Ende des ersten Schlitzabschnitts 11 geht der zweite Schlitzabschnitt 12 in radialer Richtung ab und an dessen anderem Ende befindet sich der dritte Schlitzabschnitt 13, der sich von dem zweiten Schlitzabschnitt 12 in distaler und proximaler Richtung aus erstreckt. Der erste und zweite Schlitzabschnitt 11, 12 sowie der zweite und dritte Schlitzabschnitt 12, 13 bilden zwischen sich je einen Winkel α von 90°. Eine Spiralfeder 30 drängt die Hülse 10 relativ zu der Drehwelle 20 in distaler Richtung. An der Hülse 10 und an der Drehwelle 20 ist je eine Anlagefläche 23 bzw. 110 für die Feder 30 ausgebildet, wie dies aus Fig. 1F ersichtlich ist.

Um die Betätigung der Hülse 10 zu erleichtern, sind an ihrer Außenfläche zwei ringförmige Vertiefungen 19 vorgesehen. Das in der Fig. 1 gezeigte Werkzeug 40 ist ein Spiralbohrer mit einer gehärteten Bohrerspitze 44. Dieses Ausführungsbeispiel beschreibt eine Schnellspann-Kupplung, die leicht und günstig herzustellen ist, einfach bedienbar ist und schnell und unkompliziert zerlegbar und wieder zusammenbaubar ist, um so eine gute Reinigung und Sterilisation zu gewährleisten.

Ein besonders bevorzugtes zweites Ausführungsbeispiel der vorliegenden Erfindung ist im Folgenden unter Bezugnahme auf die Fig. 1, 2F und 3B im Detail beschrieben. Die Drehwelle 20 des zweiten Ausführungsbeispiels ist identisch zu der des ersten Ausführungsbeispiels. Lediglich die Hülse 10 unterscheidet sich von der des ersten Ausführungsbeispiels. Genau genommen unterscheidet sich nur die Innenfläche der Hülse 10 und die Form des Schlitzes 11, 12, 13.

Während die Innenfläche bei dem ersten Ausführungsbeispiel eine Stufe zwischen den beiden Abschnitten 101 und 102 ausgebildet ist, ist bei diesem Ausführungsbeispiel eine geneigte Fläche 103 zwischen den beiden Abschnitten 101 und 102 vorgesehen. Der Begriff geneigt bezieht sich dabei auf die Schnittdarstellung in Fig. 3B. Die geneigte Fläche 103 ist dazu gedacht, dass die Sperrkörper, die bei diesem Ausführungsbeispiel auch Kugeln sind, sanfter in die Nut 43 in dem Werkzeug 40 gedrängt werden. Dadurch kann die Handhabung der Hülse 10 etwas angenehmer gestaltet werden, allerdings kann sich dadurch auch die erforderliche Länge der Hülse 10 erhöhen, was wiederum nicht gewünscht ist.

Der Schlitz dieses Ausführungsbeispiel ist gegenüber dem Schlitz bei dem ersten Ausführungsbeispiel in zwei Bereichen verändert. Zum einen weist der erste Schlitzabschnitt 11 bei diesem Ausführungsbeispiel eine radiale Stufe 17 auf, um ein Abspringen der Hülse von der Drehwelle 20 während der Montage oder Demontage der Schnellspann-Kupplung zu verhindern. Die radiale Stufe wird dadurch realisiert, dass in dem ersten Schlitzabschnitt 11, welcher sich bei diesem Ausführungsbeispiel größtenteils in genau distaler Richtung erstreckt, ein Bereich vorgesehen ist, in dem sich der Schlitz 11 in radialer Richtung erstreckt. Genau genommen erstreckt sich dieser Abschnitt sogar noch ein bisschen in proximaler Richtung. Dies bedeutet, dass die Stufe 17 an ihrer distalen Seite eine Art Bucht ausbildet, in der der Vorsprung 22 in Anlage geraten kann. Wenn in dieser Position des Vorsprungs 22 noch eine kleine Vorspannkraft durch die Feder 30 auf die Hülse 10 aufgebracht wird, sorgt dies dafür, dass der Vorsprung 22 in dieser Bucht an der radialen Stufe 17 quasi gesichert ist.

Zum anderen ist der dritte Schlitzabschnitt 13 stufenförmig ausgebildet. Das heißt, dass der Bereich 13A des dritten Schlitzabschnitts 13, der sich nach proximal erstreckt und somit der Schlitzabschnitt ist, in dem ggf. das Werkzeug 40 in der Werkzeugaufnahme 24 der Drehwelle 20 verriegelt ist, gegenüber dem Bereich des dritten Schlitzabschnitts 13B, der sich nach distal erstreckt und somit der Schlitzabschnitt ist, in dem ggf. das Werkzeug 40 in der Werkzeugaufnahme 24 freigegeben ist, radial versetzt ist. Zwischen den beiden Abschnitten 13A und 13B des dritten Schlitzabschnitts 13 ist ein radial verlaufender Bereich 13C vorgesehen. Der Bereich 13C ist demnach eine Verlängerung des zweiten Schlitzabschnitts 12.

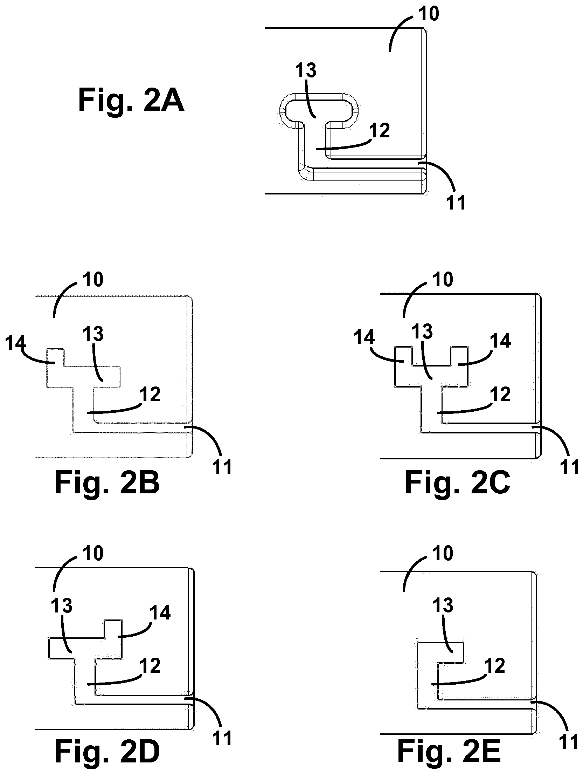

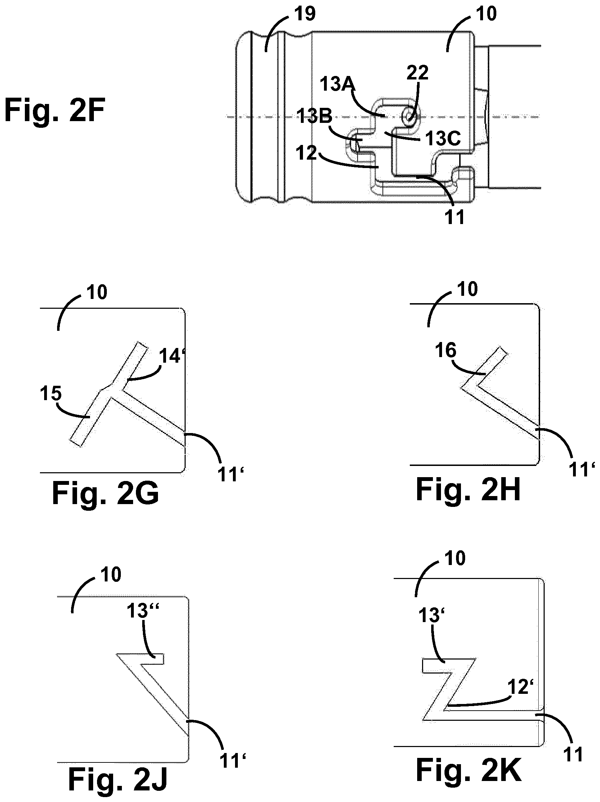

Weitere Ausführungsbeispiele der vorliegenden Erfindung sind in den Fig. 2 und 3 gezeigt. In der Fig. 2 sind zahlreiche verschiedene Formen für einen Schlitz in der Hülse 10 gezeigt. Dabei zeigen die Fig. 2A bis 2E weitere Ausführungsbeispiele, bei denen die angrenzenden Schlitzabschnitte stets einen Winkel α von 90° zwischen sich bilden und entweder in radialer oder in distaler/proximaler Richtung verlaufen. Die Fig. 2G und 2H zeigen zwei weitere Ausführungsbeispiele, bei denen die Schlitzabschnitte alle schräg bzw. diagonal verlaufen. Zudem ist in beiden Fällen der Winkel α zwischen dem ersten Schlitzabschnitt 11' und dem zweiten Schlitzabschnitt 14, 15 bzw. 16 größer als 90°. Die Fig. 2J und 2K zeigen Ausführungsbeispiele, bei denen diagonale und distale/proximale Schlitzabschnitte kombiniert sind. Auch hier sind die gebildeten Winkel α zwischen den Schlitzabschnitten deutlich größer als 90°. Auf der Basis der allgemeinen Beschreibung der verschiedenen erforderlichen und optionalen Aufgaben der Schlitzabschnitt sind diese Figuren selbsterklärend. Prinzipiell sei hier nur erwähnt, dass die Bezugszeichen 14 stets Abschnitte bezeichnen, die einer Sicherung der aktuellen Position der Hülse 10 in proximaler/distaler Richtung gegenüber der Drehwelle 20 dienen. Die verschiedenen Schlitzabschnitte der unterschiedlichen Ausführungsbeispiele der Fig. 2 können natürlich auch geeignet kombiniert werden. Beispielsweise kann sich an das proximale Ende des Schlitzabschnitts 13 gemäß der Fig. 2E noch ein radialer Schlitzabschnitt 14 entsprechend der Fig. 2D anschließen, um eine Sicherung der Hülse 10 in der Verriegelungsposition zu gewährleisten. Selbiges kann auch bei den Ausführungsbeispielen der Fig. 2G bis 2J vorgesehen werden.

In der Fig. 3B ist noch ein weiterer Aufbau der Innenfläche der Hülse 10 gezeigt. Diese Innenfläche ist mehrstufig ausgebildet und stellt somit eine Zwischenlösung zwischen den Ausführungsformen der Fig. 3A und 3C dar. Die Zwischenstufen 104 und 105 dienen dazu, die relativ große Stufe zwischen den Abschnitten 101 und 102 zu unterteilen. Je nach Bearbeitungsverfahren kann es einfacher sein, mehrere Stufen 101, 102, 104, 105 an Stelle einer geneigten Fläche 103 auszubilden, insbesondere, da diese geneigte Fläche noch in Umlaufrichtung der Hülse 10 gewölbt ist.

Alle Ausführungsformen der Innenfläche der Hülse 10 gemäß der Fig. 3 können natürlich beliebig mit allen Ausführungsformen der Schlitze in der Hülse 10 gemäß der Fig. 2 kombiniert werden. Ebenso können alle weiteren offenbarten Merkmale sinnvoll kombiniert werden.

## Patentansprüche

1. Schnellspann-Kupplung (100) für ein chirurgisches Drehwerkzeug mit einer Hülse (10), mit einer Drehwelle (20), und mit einem elastischen Element (30),
wobei in der distaler Endfläche der Drehwelle (20) eine Werkzeugaufnahme (24) ausgebildet ist, welche daran angepasst ist, ein chirurgisches Drehwerkzeug drehfest aufzunehmen, wobei die Drehwelle (20) im Bereich ihres distalen Endes (21) mindestens einen radialen Vorsprung (22) und mindestens eine radiale Durchbrechung aufweist, wobei in der mindestens einen Durchbrechung ein Sperrkörper (25) angeordnet ist, welcher in radialer Richtung zu der Drehwelle (20) bewegbar ist,
wobei die Hülse (10) mit ihrem proximalen Ende über das distale Ende (21) der Drehwelle (20) schiebbar ist, wobei die Hülse (10) mindestens einen Schlitz (11, 11', 12, 12', 13, 13', 13", 14, 15, 16, 17) aufweist, der einen ersten Schlitzabschnitt (11, 11') und wenigstens einen weiteren Schlitzabschnitt (12, 12', 13, 13', 13", 14, 15, 16, 17) aufweist,
wobei der weitere Schlitzabschnitt (12, 12', 13, 13', 13", 14, 15, 16, 17) mit dem ersten Schlitzabschnitt (11, 11') in Verbindung steht, und das elastische Element (30) die Hülse (10) relativ zu der Drehwelle (20) in distaler Richtung drängt, **dadurch gekennzeichnet, dass** sich der erste Schlitzabschnitt (11, 11') vom proximalen Ende der Hülse (10) aus erstreckt, sodass der radiale Vorsprung (22) von dem proximalen Ende der Hülse (10) in den Schlitz (11, 11', 12, 12', 13, 13', 13", 14, 15, 16, 17) einführbar ist,
und dass die Innenfläche der Hülse (10) einen Bereich aufweist, in dem der Innendurchmesser des proximalen Abschnitts (102) kleiner als der Innendurchmesser des distalen Abschnitts (101) ist, wobei der Bereich daran angepasst ist, bei einer Bewegung der Hülse (10) in distaler Richtung in Anlage an den mindestens einen Sperrkörper (25) zu gelangen und ihn zur Achse der Drehwelle (20) hin zu drängen und bei einer Bewegung der Hülse (10) in proximaler Richtung der mindestens eine Sperrkörper (25) frei gegeben wird, sodass sich dieser zumindest so weit radial nach außen bewegen kann, dass dessen inneres Ende aus der Werkzeugaufnahme (24) austritt.

2. Schnellspann-Kupplung (100) nach Anspruch 1, wobei
das elastische Element (30) eine Spiralfeder ist, welche um die Drehwelle (20) herum angeordnet ist.

3. Schnellspann-Kupplung (100) nach einem der Ansprüche 1 oder 2, wobei an der Hülse (10) und/oder an der Drehwelle (20) eine Anlagefläche (23, 110) für das elastische Element (30) ausgebildet ist.

4. Schnellspann-Kupplung (100) nach einem der vorangehenden
Ansprüche, wobei
der Sperrkörper eine Kugel ist und die Durchbrechung an beiden Seiten einen gegenüber einem dazwischen liegenden Abschnitt verkleinerten Durchmesser aufweist.

5. Schnellspann-Kupplung (100) nach einem der vorangehenden
Ansprüche, wobei
sich der erste Schlitzabschnitt (11) im Wesentlichen in distaler Richtung erstreckt und an seinem distalen Ende mit dem zweiten Schlitzabschnitt (12) in Verbindung steht, der sich im Wesentlichen in radialer Richtung erstreckt.

6. Schnellspann-Kupplung (100) nach einem der Ansprüche 1 bis 4, wobei
sich der erste Schlitzabschnitt (11') schräg in distaler und radialer Richtung erstreckt und an seinem distalen Ende mit dem zweiten Schlitzabschnitt (13", 14, 15, 16) in Verbindung steht.

7. Schnellspann-Kupplung (100) nach Anspruch 6, wobei
sich der zweite Schlitzabschnitt (13", 14, 15, 16) in proximaler Richtung erstreckt und vorzugsweise in proximaler und radialer Richtung erstreckt.

8. Schnellspann-Kupplung (100) nach einem der Ansprüche 5 bis 7, wobei
der erste Schlitzabschnitt (11, 11') über den zweiten Schlitzabschnitt (12, 12') mit einem dritten Schlitzabschnitt (13, 13') in Verbindung steht, welcher sich von dem zweiten Schlitzabschnitt (12, 12') im Wesentlichen in distaler und/oder proximaler Richtung erstreckt.

9. Schnellspann-Kupplung (100) nach Anspruch 6, wobei
ein Ende des dritten Schlitzabschnitts (13, 13') mit einem vierten Schlitzabschnitt (14) in Verbindung steht, der sich im Wesentlichen in radialer Richtung erstreckt, wobei vorzugsweise das andere Ende des dritten Schlitzabschnitts (13, 13') mit einem fünften Schlitzabschnitt (14) in Verbindung steht, der sich im Wesentlichen in radialer oder gegenradialer Richtung erstreckt.

10. Schnellspann-Kupplung (100) nach einem der vorangehenden
Ansprüche, wobei
im ersten Schlitzabschnitt (11, 11') in der Nähe des proximalen Randes der Hülse (10) eine radiale Stufe (17) ausgebildet ist.

11. Schnellspann-Kupplung (100) nach einem der vorangehenden
Ansprüche, wobei
die Schlitzabschnitte (11, 11', 12, 12', 13, 13', 13", 14, 15, 16, 17) auch gebogen oder gekrümmt ausgebildet sein können.

12. Schnellspann-Kupplung (100) nach einem der vorangehenden
Ansprüche, wobei
zwei oder drei Schlitze vorgesehen sind, die in gleichwinkligen Abständen entlang der Umfangsrichtung der Hülse (10) angeordnet sind, und wobei
eine gleiche Anzahl von radialen Vorsprüngen (22) an der Drehwelle (20) ausgebildet sind, die entsprechend angeordnet sind.

13. Schnellspann-Kupplung (100) nach einem der vorangehenden
Ansprüche, wobei
zwei oder drei radiale Durchbrechungen in der Seitenwand der Werkzeugaufnahme (24) ausgebildet sind und in jeder Durchbrechung eine Kugel als Sperrkörper angeordnet ist, welche in der Durchbrechung gehalten wird, indem zumindest ein Teil wenigstens eines Rands der Durchbrechung zur Lochmitte hin plastisch verformt ist.

14. Schnellspann-Kupplung (100) nach einem der vorangehenden
Ansprüche, wobei
in dem Bereich der Innenfläche der Hülse (10) zwischen dem proximalen Abschnitt (101) und dem distalen Abschnitt (102) eine Stufe (104, 105) oder eine geneigte Fläche (103) vorgesehen ist.

15. Schnellspann-Kupplung (100) nach einem der vorangehenden
Ansprüche, wobei
der Schlitz derart ausgestaltet ist, dass er sich über die gesamte Wanddicke der Hülse (10) erstreckt.

## Claims

1. Quick-action coupling (100) for a rotary surgical tool, having a sleeve (10), having a rotary shaft (20), and having an elastic element (30), wherein in the distal end surface of the rotary shaft (20) there is formed a tool receptacle (24) which is adapted to receive a rotary surgical tool for conjoint rotation therewith, wherein the rotary shaft (20) has, in the area of its distal end (21), at least one radial projection (22) and at least one radial aperture, wherein a locking body (25) is arranged in the at least one aperture, said locking body being movable in the radial direction with respect to the rotary shaft (20), wherein the proximal end of the sleeve (10) is able to be pushed over the distal end (21) of the rotary shaft (20), wherein the sleeve (10) has at least one slot (11, 11', 12, 12', 13, 13', 13'', 14, 15, 16, 17) which has a first slot portion (11, 11') and at least one further slot portion (12, 12', 13, 13', 13'', 14, 15, 16, 17), wherein the further slot portion (12, 12', 13, 13', 13'', 14, 15, 16, 17) is connected to the first slot portion (11, 11'), and the elastic element (30) urges the sleeve (10) in the distal direction relative to the rotary shaft (20), **characterized in that** the first slot portion (11, 11') extends from the proximal end of the sleeve (10), such that the radial projection (22) can be inserted from the proximal end of the sleeve (10) into the slot (11, 11', 12, 12', 13, 13', 13", 14, 15, 16, 17), and **in that** the inner surface of the sleeve (10) has an area in which the internal diameter of the proximal portion (102) is smaller than the internal diameter of the distal portion (101), wherein the area is adapted to come to bear on the at least one locking body (25) during a movement of the sleeve (10) in the distal direction and to urge the locking body toward the axis of the rotary shaft (20) and, in the case of a movement of the sleeve (10) in the proximal direction, the at least one locking body (25) is freed, such that it can move radially outward at least to the extent that its inner end emerges from the tool receptacle (24).

2. Quick-action coupling (100) according to Claim 1, wherein the elastic element (30) is a helical spring which is arranged about the rotary shaft (20) .

3. Quick-action coupling (100) according to either of Claims 1 and 2, wherein a bearing surface (23, 110) for the elastic element (30) is formed on the sleeve (10) and/or on the rotary shaft (20).

4. Quick-action coupling (100) according to one of the preceding claims, wherein the locking body is a ball, and the aperture has, on both sides, a diameter that is smaller compared to a portion lying between said sides.

5. Quick-action coupling (100) according to one of the preceding claims, wherein the first slot portion (11) extends substantially in the distal direction and, at its distal end, is connected to the second slot portion (12), which extends substantially in the radial direction.

6. Quick-action coupling (100) according to one of Claims 1 to 4, wherein the first slot portion (11') extends obliquely in the distal and radial direction and, at its distal end, is connected to the second slot portion (13'', 14, 15, 16).

7. Quick-action coupling (100) according to Claim 6, wherein the second slot portion (13'', 14, 15, 16) extends in the proximal direction and preferably extends in the proximal and radial direction.

8. Quick-action coupling (100) according to one of Claims 5 to 7, wherein the first slot portion (11, 11') is connected via the second slot portion (12, 12') to a third slot portion (13, 13'), which extends from the second slot portion (12, 12') substantially in the distal and/or proximal direction.

9. Quick-action coupling (100) according to Claim 6, wherein one end of the third slot portion (13, 13') is connected to a fourth slot portion (14), which extends substantially in the radial direction, wherein the other end of the third slot portion (13, 13') is preferably connected to a fifth slot portion (14), which extends substantially in the radial or contra-radial direction.

10. Quick-action coupling (100) according to one of the preceding claims, wherein a radial step (17) is formed in the first slot portion (11, 11'), near the proximal edge of the sleeve (10).

11. Quick-action coupling (100) according to one of the preceding claims, wherein the slot portions (11, 11', 12, 12', 13, 13', 13'', 14, 15, 16, 17) can also have a bent or curved shape.

12. Quick-action coupling (100) according to one of the preceding claims, wherein two or three slots are provided which are arranged at uniform angular intervals along the circumferential direction of the sleeve (10), and wherein an equal number of radial projections (22) are formed on the rotary shaft (20) and are arranged correspondingly.

13. Quick-action coupling (100) according to one of the preceding claims, wherein two or three radial apertures are formed in the side wall of the tool receptacle (24) and a ball is arranged as a locking body in each aperture, said ball being held in the aperture by means of at least one part of at least one edge of the aperture being plastically deformed toward the hole center.

14. Quick-action coupling (100) according to one of the preceding claims, wherein a step (104, 105) or an inclined surface (103) is provided in the area of the inner surface of the sleeve (10) between the proximal portion (101) and the distal portion (102) .

15. Quick-action coupling (100) according to one of the preceding claims, wherein the slot is configured in such a way that it extends through the entire wall thickness of the sleeve (10).

## Revendications

1. Raccord à serrage rapide (100) pour un instrument chirurgical rotatif avec un manchon (10), avec un arbre rotatif (20) et avec un élément élastique (30), dans lequel un logement d'instrument (24) est pratiqué dans la face d'extrémité distale de l'arbre rotatif (20) et est adapté pour recevoir sans rotation un instrument chirurgical rotatif, dans lequel l'arbre rotatif (20) présente dans la région de son extrémité distale (21) au moins une saillie radiale (22) et au moins une percée radiale, dans lequel un corps d'arrêt (25), qui est déplaçable en direction radiale par rapport à l'arbre rotatif (20), est disposé dans ladite au moins une percée, dans lequel le manchon (10) peut être glissé avec son extrémité proximale sur l'extrémité distale (21) de l'arbre rotatif (20), dans lequel le manchon (10) présente au moins une fente (11, 11', 12, 12', 13, 13', 13", 14, 15, 16, 17), qui présente une première partie de fente (11, 11') et au moins une autre partie de fente (12, 12', 13, 13', 13", 14, 15, 16, 17), dans lequel l'autre partie de fente (12, 12', 13, 13', 13", 14, 15, 16, 17) est en communication avec la première partie de fente (11, 11'), et l'élément élastique (30) repousse le manchon (10) en direction distale par rapport à l'arbre rotatif (20),
**caractérisé en ce que** la première partie de fente (11, 11') s'étend à partir de l'extrémité proximale du manchon (10), de telle manière que la saillie radiale (22) puisse être introduite dans la fente (11, 11', 12, 12', 13, 13', 13", 14, 15, 16, 17) par l'extrémité proximale du manchon (10), et **en ce que** la face intérieure du manchon (10) présente une zone dans laquelle le diamètre intérieur de la partie proximale (102) est plus petit que le diamètre intérieur de la partie distale (101), dans lequel la zone est adaptée pour, lors d'un mouvement du manchon (10) en direction distale, venir buter contre ledit au moins un corps d'arrêt (25) et le repousser en direction de l'axe de l'arbre rotatif (20) et, lors d'un mouvement du manchon (10) en direction proximale, libérer ledit au moins un corps d'arrêt (25), de telle manière que celui-ci puisse se déplacer radialement vers l'extérieur au moins à un point tel que son extrémité intérieure sorte du logement d'instrument (24).

2. Raccord à serrage rapide (100) selon la revendication 1, dans lequel l'élément élastique (30) est un ressort spiral, qui est disposé autour de l'arbre rotatif (20).

3. Raccord à serrage rapide (100) selon une des revendications 1 ou 2, dans lequel une face d'appui (23, 110) pour l'élément élastique (30) est formée sur le manchon (10) et/ou sur l'arbre rotatif (20).

4. Raccord à serrage rapide (100) selon l'une quelconque des revendications précédentes, dans lequel le corps d'arrêt est une bille et la percée présente sur les deux côtés un diamètre réduit par rapport à une partie située entre ceux-ci.

5. Raccord à serrage rapide (100) selon l'une quelconque des revendications précédentes, dans lequel la première partie de fente (11) s'étend essentiellement en direction distale et est en communication à son extrémité distale avec la deuxième partie de fente (12), qui s'étend essentiellement en direction radiale.

6. Raccord à serrage rapide (100) selon l'une quelconque des revendications 1 à 4, dans lequel la première partie de fente (11') s'étend en oblique en direction distale et radiale et est en communication à son extrémité distale avec la deuxième partie de fente (13", 14, 15, 16).

7. Raccord à serrage rapide (100) selon la revendication 6, dans lequel la deuxième partie de fente (13", 14, 15, 16) s'étend en direction proximale et s'étend de préférence en direction proximale et radiale.

8. Raccord à serrage rapide (100) selon l'une quelconque des revendications 5 à 7, dans lequel la première partie de fente (11, 11') est en communication par la deuxième partie de fente (12, 12') avec une troisième partie de fente (13, 13'), qui s'étend à partir de la deuxième partie de fente (12, 12') essentiellement en direction distale et/ou proximale.

9. Raccord à serrage rapide (100) selon la revendication 6, dans lequel une extrémité de la troisième partie de fente (13, 13') est en communication avec une quatrième partie de fente (14), qui s'étend essentiellement en direction radiale, dans lequel de préférence l'autre extrémité de la troisième partie de fente (13, 13') est en communication avec une cinquième partie de fente (14), qui s'étend essentiellement en direction radiale ou contre-radiale.

10. Raccord à serrage rapide (100) selon l'une quelconque des revendications précédentes, dans lequel un gradin radial (17) est formé dans la première partie de fente (11, 11') à proximité du bord proximal du manchon (10) .

11. Raccord à serrage rapide (100) selon l'une quelconque des revendications précédentes, dans lequel les parties de fente (11, 11', 12, 12', 13, 13', 13", 14, 15, 16, 17) peuvent également être de forme courbée ou incurvée.

12. Raccord à serrage rapide (100) selon l'une quelconque des revendications précédentes, dans lequel il est prévu deux ou trois fentes, qui sont disposées à des distances angulaires égales le long de la direction périphérique du manchon (10), et dans lequel un nombre identique de saillies radiales (22) sont formées sur l'arbre rotatif (20) et sont disposées de façon correspondante.

13. Raccord à serrage rapide (100) selon l'une quelconque des revendications précédentes, dans lequel deux ou trois percées sont formées dans la paroi latérale du logement d'instrument (24) et une bille est disposée en tant que corps d'arrêt dans chaque percée, laquelle est maintenue dans la percée du fait qu'au moins une partie d'au moins un bord de la percée est déformée plastiquement en direction du milieu du trou.

14. Raccord à serrage rapide (100) selon l'une quelconque des revendications précédentes, dans lequel il est prévu un gradin (104, 105) ou une face inclinée (103) dans la région de la face intérieure du manchon (10) entre la partie proximale (101) et la partie distale (102) .

15. Raccord à serrage rapide (100) selon l'une quelconque des revendications précédentes, dans lequel la fente est configurée de telle manière qu'elle s'étende sur toute l'épaisseur de paroi du manchon (10).
